# EUROPEAN PATENT SPECIFICATION

(11) **EP 0 925 070 B1**
(45) Date of publication and mention of the grant of the patent: **03.09.2003**
(21) Application number: 97941495.0
(22) Date of filing: 09.09.1997
(51) Int. Cl.: A61P 31/10, A61K 38/12, A61K 45/06

(54) **ANTIFUNGAL COMBINATION THERAPY**
FUNGIZIDE KOMBINATIONSTHERAPIE
THERAPIE ANTIFONGIQUE COMBINEE

(30) Priority: 12.09.1996 US 26327 P; 04.10.1996 GB 9620739
(43) Date of publication of application: 30.06.1999
(73) Proprietor: MERCK & CO., INC., Rahway, New Jersey 07065 (US)
(72) Inventor: BARTIZAL, Kenneth, F., Rahway, NJ 07065 (US); ABRUZZO, George, K., Rahway, NJ 07065 (US); FLATTERY, Amy, M., Rahway, NJ 07065 (US)
(74) Representative: Thompson, John Dr.
(86) International application number: US9715987
(87) International publication number: WO98010782

(56) References cited:
- WO-A-94/21677
- K. BARTIZAL ET AL.: "IN VITRO EVALUATION OF THE PNEUMOCANDIN ANTIFUNGAL AGENT L-733560, A NEW WATER-SOLUBLE HYBRID OF L-705589 AND L-731373." ANTIMICROBIAL AGENTS AND CHEMOTHERAPY, vol. 39, no. 5, May 1995, BETHESDA, MD, US, pages 1070-1076, XP002051763 cited in the application
- S.P. FRANZOT ET AL.: "PNEUMOCANDIN L-743872 ENHANCES THE ACTIVITIES OF AMPHOTERICIN B AND FLUCONAZOLE AGAINST CRYPTOCOCCUS NEOFORMANS IN VITRO." ANTIMICROBIAL AGENTS AND CHEMOTHERAPY, vol. 41, no. 2, February 1997, BETHESDA, MD, US, pages 331-336, XP002051764

## Description

### FIELD OF THE INVENTION

The present invention relates to antifungal combination therapy comprising the use of known antifungal agents such as the azoles or polyenes in combination with a pneumocandin derivative antifungal agent. More particularly, the invention relates to antifungal combination therapy comprising the use of azoles such as fluconazole (hereinafter referred to as FCZ), voriconazole, itraconazole, ketoconazole, miconazole, ER 30346, SCH 56592; polyenes such as amphotericin B (hereinafter referred to as AmB), nystatin or liposomal and lipid forms thereof such as Abelcet, AmBisome and Amphocil; purine or pyrimidine nucleotide inhibitors such as flucytosine; or polyoxins such as nikkomycins, in particular nikkomycin Z or other chitin inhibitors, elongation factor inhibitors such as sordarin and analogs thereof, mannan inhibitors such as predamycin , bactericidal/permeability-inducing (BPI) protein products such as XMP.97 or XMP.127 or complex carbohydrate antifungal agents such as CAN-296 in combination with a pneumocandin derivative of the structure 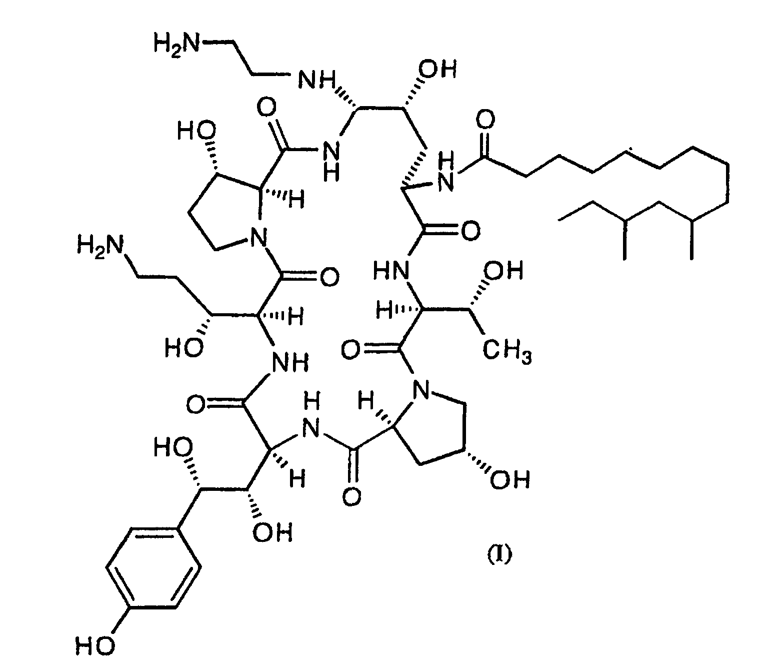 or a pharmaceutically acceptable salt or other pharmaceutically acceptable formulation thereof.

These combination therapies have been shown to be useful against such opportunistic pathogens as *Cryptococcus* spp., *Candida* spp., *Aspergillus* spp., *Histoplasma* spp., *Coccidioides* spp., *Paracoccidioides* spp. *Blastomyces* spp., *Fusarium* spp., *Sporothrix* spp., *Trichosporon* spp., *Rhizopus* spp., *Pseudallescheria* spp., dermatophytes, *Paeciliomyces* spp., *Alternaria* spp., *Curvularia* spp., *Exophiala* spp., *Wangiella* spp., *Penicillium* spp., *Saccharomyces* spp., *Dematiaceous* fungi and *Pneumocystis carinii*.

### BACKGROUND OF THE INVENTION

There is an increasing need for agents which are effective against opportunistic mycotic infections by such agents as *Cryptococcus* spp., *Candida* spp., *Aspergillus* spp., *Histoplasma* spp., *Coccidioides* spp., *Paracoccidioides* spp. *Blastomyces* spp., *Fusarium* spp., *Sporothrix* spp., *Trichosporon* spp., *Rhizopus* spp., *Pseudallescheria* spp., dermatophytes, *Paeciliomyces* spp., *Alternaria* spp., *Curvularia* spp., *Exophiala* spp., *Wangiella* spp., *Penicillium* spp., *Saccharomyces* spp., *Dematiaceous* fungi and *Pneumocystis carinii*. The present treatments, i.e., polyenes, such as amphotericin B, cause severe side effects and azoles, such as fluconazole, are only fungistatic. The pneumocandins, which are related to the echinocandins, are cyclic hexapeptides which inhibit cell wall 1,3β-D-glucan synthesis. The pneumocandins have shown potent *in vivo* activity against *Candida* spp., *Pneumocystis carinii, Aspergillus* spp., as well as the other fungal pathogens listed above. However, the pneumocandins, by themselves, have weak activity against *Cryptococcus* spp.

Combination therapy with antifungal drugs may provide additional options for treating *Cryptococcus* and other fungal pathogens.

Previous studies have evaluated the efficacy of other pneumocandin derivatives against *Cryptococcus neoformans* in combination with amphotericin B and fluconazole (Abruzzo et al., Antimicrob. Agents Chemo. 1995, 39:1077-1081 and Bartizal et al., Antimicrob. Agents Chemo. 1995, 39:1070-1076). However, none of these studies have demonstrated the results found using Compound I as the pneumocandin derivative.

### DETAILED DESCRIPTION OF THE INVENTION

The present invention relates to antifungal combination therapy comprising the use of known antifungal agents such as the azoles or polyenes in combination with a pneumocandin derivative antifungal agent. More particularly, the invention relates to antifungal combination therapy comprising the use of azoles such as fluconazole, voriconazole, itraconazole, ketoconazole, miconazole, ER 30346, SCH 56592; polyenes such as amphotericin B, nystatin or liposomal and lipid forms thereof such as Abelcet, AmBisome and Amphocil; purine or pyrimidine nucleotide inhibitors such as flucytosine; or polyoxins such as nikkomycins, in particular nikkomycin Z or other chitin inhibitors, elongation factor inhibitors such as sordarin and analogs thereof, mannan inhibitors such as predamycin , bactericidal/permeability- inducing (BPI) protein products such as XMP.97 or XMP.127 or complex carbohydrate antifungal agents such as CAN-296 in combination with a compound of the structure 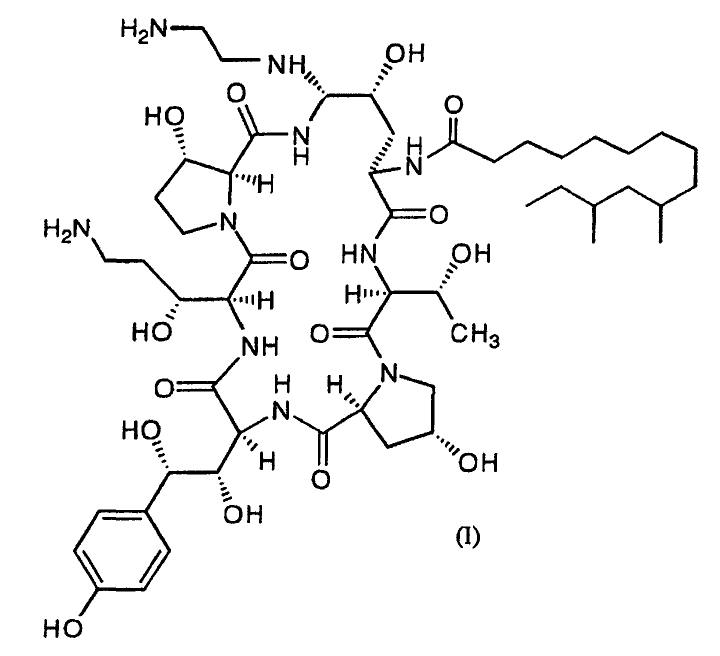 or a pharmaceutically acceptable salt or other pharmaceutically acceptable formulation thereof.

In particular, this combination therapy has been shown to be useful against such opportunistic pathogens as *Cryptococcus* spp., *Candida* spp., *Aspergillus* spp., *Histoplasma* spp., *Coccidioides* spp., *Paracoccidioides* spp. *Blastomyces* spp., *Fusarium* spp., *Sporothrix* spp., *Trichosporon* spp., *Rhizopus* spp., *Pseudallescheria* spp., dermatophytes, *Paeciliomyces* spp., *Alternaria* spp., *Curvularia* spp., *Exophiala* spp., *Wangiella* spp., *Penicillium* spp., *Saccharomyces* spp., *Dematiaceous* fungi and *Pneumocystis carinii*.

Compound I is disclosed in U.S. Patent No. 5,378,804. Its preparation is described in that patent along with U.S. Patent No. 5,552,521.

It is contemplated that other pneumocandin derivatives such as those disclosed in U.S. Patent No. 5,516,756 and in copending applications 07/936,558, 07/936,561, 08/058,657, 08/055,996, 08/378,687 and 60/006,505 would be useful in the combination therapy.

The azole, polyene or other antifungal agent may be administered orally or parenterally. Compound I is preferably administered parenterally, but is not limited to that route and may also be administered by other routes such as oral, intramuscular or subcutaneous.

As shown below, the combination therapy results in enhanced effects using sub-inhibitory concentrations of all agents. These effects can be demonstrated *in vitro* and *in vivo* using clinical and environmental strains of *C. neoformans, C. albicans* and *A. fumigatus*.

The invention is further described in connection with the following non-limiting examples.

### EXAMPLES

It has been found that combination therapy of Compound I with AmB and FCZ against *C. neoformans* results in enhanced activity against strains of *C. neoformans in vitro.* It has also been found that combination therapy of Compound I with AmB against *C. albicans* and *A. fumigatus* results in enhanced activity *in vitro.* This has been shown using a broth microdilution technique which is the standard method for antifungal susceptibility testing proposed by the NCCLS (protocol M27-T). Sub-inhibitory concentrations of Compound I in combination with sub-inhibitory concentrations of AmB and sub-inhibitory concentrations of FCZ were employed. The minimal inhibitory concentrations (MICs) for AmB and Compound I were defined as the lowest drug concentration at which there was an absence of growth. FCZ MIC was defined as the lowest drug concentration which resulted in a visual turbidity less than or equal to 80% inhibition compared with that produced by the control without antifungal agent.

Results of antifungal susceptibility testing show that colony forming units (CFUs) were markedly reduced when amphotericin B at certain concentrations (0.0075, 0.015 and 0.03 µg/ml) was combined with Compound I at certain concentrations (4, 8 and 16 µg/ml). Additionally, the administration of Compound I significantly enhanced the activity of fluconazole by reducing CFU numbers at certain concentrations (0.25, 0.50 and 1.0 µg/ml).

Additional drug combination testing *in vitro* was performed to evaluate combinations of Compound I with AmB and FCZ against clinical isolates. There was no antagonism evident between Compound I and AmB against *C. albicans, A. fumigatus* and *C. neoformans*. Fractional inhibitory indices (FIC) were approximately 0.50 or lower, indicative of additive or synergistic activity. Results suggest that Compound I can enhance the activity of FCZ and AmB and indicate a potential role for Compound I in combination regimes against those fungi less sensitive or insensitive to Compound I when used alone.

Drug interaction and efficacy studies with Compound I combined with either AmB or FCZ against disseminated candidiasis, aspergillosis and cryptococcosis were performed. Results showed no adverse effects with combinations at high, use or lower concentrations and no antagonism of efficacy with either AmB or FCZ combined with Compound I. Against *C. albicans*, Compound I doses of 0.03 mg/kg and lower plus 0.03 mg/kg and lower of AmB appeared more efficacious than either agent administered alone. With FCZ similar results were found at doses of 0.31 mg/kg and lower of FCZ when combined with 0.03 mg/kg of Compound I. Against *A. fumigatus*, significant improvements in efficacy (10 to >800-fold in ED values) with combinations were noted with Compound I titrated between 0.03 and 2 mg/kg and with AmB between 0.03 and 0.5 mg/kg. Significant improvement in survival was seen with Compound I at 0.008 mg/kg combined with AmB at 0.12 mg/kg over the compounds administered alone.

As found in drug combination studies *in vitro* with Compound I and AmB, FIC values were approximately 0.50, suggesting additive or synergistic activity *in vivo*. Additionally, no antagonism of efficacy with FCZ in combination with Compound I was seen in studies against cryptococcosis in mice. Beneficial effects on efficacy were observed against *C. neoformans* with combinations of Compound I and FCZ at certain concentrations.

## Claims

1. Use of a pneumocandin derivative and another antifungal agent selected from the group consisting of azoles, polyenes, purine nucleotide inhibitors, pyrimidine nucleotide inhibitors, mannan inhibitors, protein elongation factor inhibitors, bactericidal/permeability inducing protein products or polyoxins in the manufacture of a medicament for treating fungal infections, wherein the pneumocandin derivative is 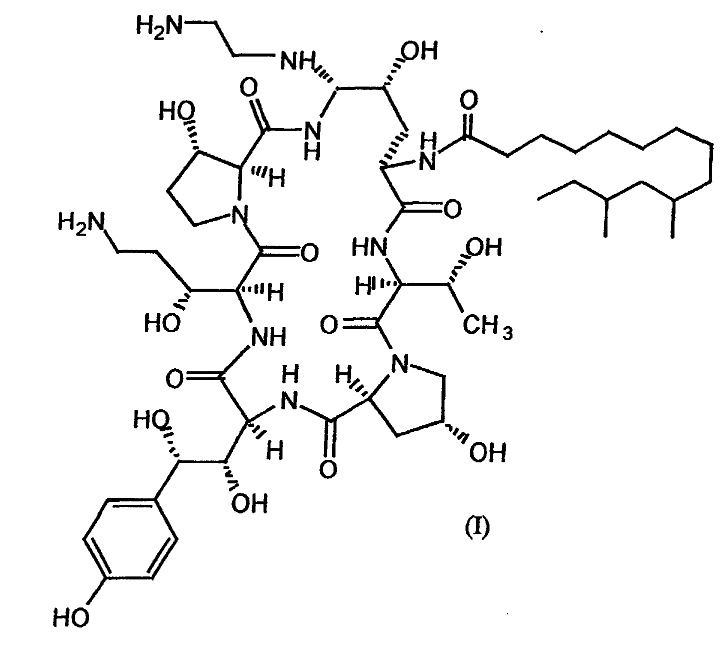 or a pharmaceutically acceptable salt thereof.

2. The use as claimed in Claim 1 wherein the pneumocandin derivative is formulated for parenteral administration.

3. The use as claimed in Claim 1 or Claim 2 wherein the azole, polyene or other antifungal agent is formulated for oral or parenteral administration.

4. The use as claimed in any one of Claims 1 to 3 wherein the azole is selected from the group consisting of fluconazole, voriconazole, itraconazole, ketoconazole, miconazole, ER 30346, and SCH *56592;* the polyene is selected from the group consisting of amphotericin B, nystatin or liposomal and lipid forms thereof; the purine or pyrimidine nucleotide inhibitor is flucytosine; the mannan inhibitor is predamycin; the protein elongation factor inhibitor is sordarin and analogs thereof; and the polyoxin is nikkomycin Z.

5. The use as claimed in any one of Claims 1 to 3 wherein the other antifungal agent is a polyene.

6. The use as claimed in any one of Claims 1 to 3 wherein the other antifungal agent is an azole.

7. The use as claimed in Claim 5 wherein the polyene is amphotericin B.

8. The use as claimed in Claim 6 wherein the azole is fluconazole.

9. The use as claimed in any one of Claims 1 to 8 wherein the infection is caused by a fungal pathogen selected from *Cryptococcus* spp., *Candida* spp., *Aspergillus* spp., *Histoplasma* spp., *Coccidioides* spp., *Paracoccidioides* spp. *Blastomyces* spp., *Fusarium* spp., *Sporothrix* spp., *Trichosporon* spp., *Rhizopus* spp., *Pseudallescheria* spp., dermatophytes, *Paeciliomyces* spp., *Alternaria* spp., *Curvularia* spp., *Exophiala* spp., *Wangiella* spp., *Penicillium* spp., *Saccharomyces* spp., *Dematiaceous.* fungi or *Pneumocystis carinii*.

10. The use as claimed in Claim 9 wherein the fungal pathogen is selected from *Cryptococcus* spp., *Candida* spp. or *Aspergillus* spp.

11. The use as claimed in Claim 7 or Claim 8 wherein the fungal pathogen is a clinical or environmental strain of *Cryptococcus neoformans*.

12. The use as claimed in Claim 7 wherein the fungal pathogen is a clinical or environmental strain of *Candida albicans* or *Aspergillus fumigatus.*

13. The use as claimed in Claim 6 wherein the azole is SCH 56592.

## Patentansprüche

1. Die Verwendung eines Pneumocandin-Derivats und eines weiteren antifungalen Mittels, ausgewählt aus der Gruppe, bestehend aus Azolen, Polyenen, Purinnukleotidinhibitoren, Pyrimidinnukleotidinhibitoren, Mannaninhibitoren, Proteinelongationsfaktorinhibitoren, bakteriziden/permeabilitätsinduzierenden Eiweißprodukten oder Polyoxinen, bei der Herstellung eines Medikaments zur Behandlung von Pilzinfektionen, wobei das Pneumocandin-Derivat 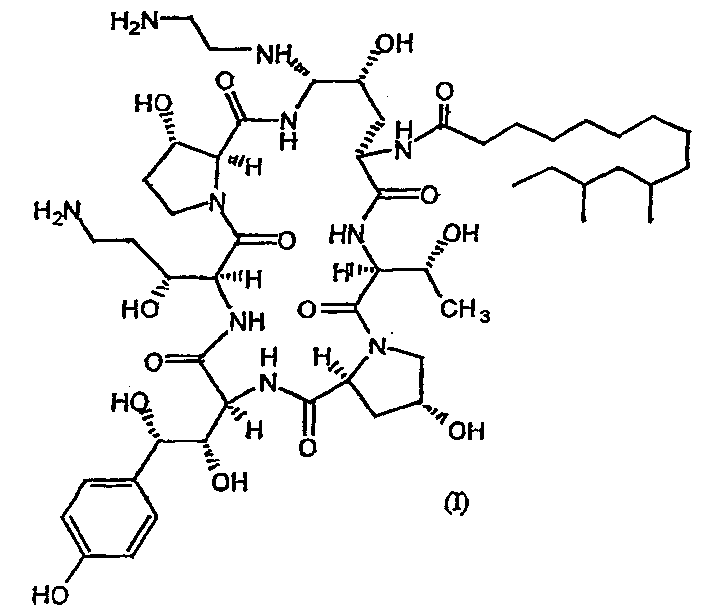 oder ein pharmazeutisch annehmbares Salz davon ist.

2. Die wie in Anspruch 1 beanspruchte Verwendung, wobei das Pneumocandin-Derivat zur parenteralen Verabreichung formuliert ist.

3. Die wie in Anspruch 1 oder Anspruch 2 beanspruchte Verwendung, wobei das Azol, Polyen oder andere antifungale Mittel zur oralen oder parenteralen Verabreichung formuliert ist.

4. Die wie in irgendeinem der Ansprüche 1 bis 3 beanspruchte Verwendung, wobei das Azol ausgewählt ist aus der Gruppe, bestehend aus Fluconazol, Voriconazol, Itraconazol, Ketoconazol, Miconazol, ER 30346 und SCH 56592, das Polyen ausgewählt ist aus der Gruppe, bestehend aus Amphotericin B, Nystatin oder liposomalen und Lipidformen davon, der Purin- oder Pyrimidinnukleotidinhibitor Flucytosin ist, der Mannaninhibitor Predamycin ist, der Proteinelongationsfaktorinhibitor Sordarin und Analoga davon ist und das Polyoxin Nikkomycin Z ist.

5. Die wie in irgendeinem der Ansprüche 1 bis 3 beanspruchte Verwendung, wobei das andere antifungale Mittel ein Polyen ist.

6. Die wie in irgendeinem der Ansprüche 1 bis 3 beanspruchte Verwendung, wobei das andere antifungale Mittel ein Azol ist.

7. Die wie in Anspruch 5 beanspruchte Verwendung, wobei das Polyen Amphotericin B ist.

8. Die wie in Anspruch 6 beanspruchte Verwendung, wobei das Azol Fluconazol ist.

9. Die wie in irgendeinem der Ansprüche 1 bis 8 beanspruchte Verwendung, wobei die Infektion von einem fungalen Krankheitserreger, ausgewählt aus *Cryptococcus* spp., *Candida* spp., *Aspergillus* spp., *Histoplasma* spp., *Coccidioides* spp., *Paracoccidioides* spp., *Blastomyces* spp., *Fusarium* spp., *Sporothrix* spp., *Trichosporon* spp., *Rhizopus* spp., *Pseudallescheria* spp., Dermatophytes, *Paeciliomyces* spp., *Alternaria* spp., *Curvularia* spp., *Exophiala* spp., *Wangiella* spp., *Penicillium* spp., *Saccharomyces* spp., *Dematiaceous*-Pilz oder *Pneumocystis carinii*, verursacht wird.

10. Die wie in Anspruch 9 beanspruchte Verwendung, wobei der fungale Krankheitserreger ausgewählt ist aus *Cryptococcus* spp., *Candida* spp. oder *Aspergillus* spp.

11. Die wie in Anspruch 7 oder Anspruch 8 beanspruchte Verwendung, wobei der fungale Krankheitserreger ein klinischer oder Umweltstamm von *Cryptococcus neoformans* ist.

12. Die wie in Anspruch 7 beanspruchte Verwendung, wobei der fungale Krankheitserreger ein klinischer oder Umweltstamm von *Candida albicans* oder *Aspergillus fumigatus* ist.

13. Die wie in Anspruch 6 beanspruchte Verwendung, wobei das Azol SCH 56592 ist.

## Revendications

1. Utilisation d'un dérivé de la pneumocandine et d'un autre agent anti-fongique choisi dans le groupe constitué par les azoles, les polyènes, les inhibiteurs des nucléotides puriques, les inhibiteurs des nucléotides pyrimidiques, les inhibiteurs du mannane, les inhibiteurs du facteur d'élongation des protéines, des produits protéiques ou des polyoxines induisant une perméabilité bactéricide, dans la fabrication d'un médicament pour traiter des infections fongiques, dans laquelle le dérivé de la pneumocandine est : 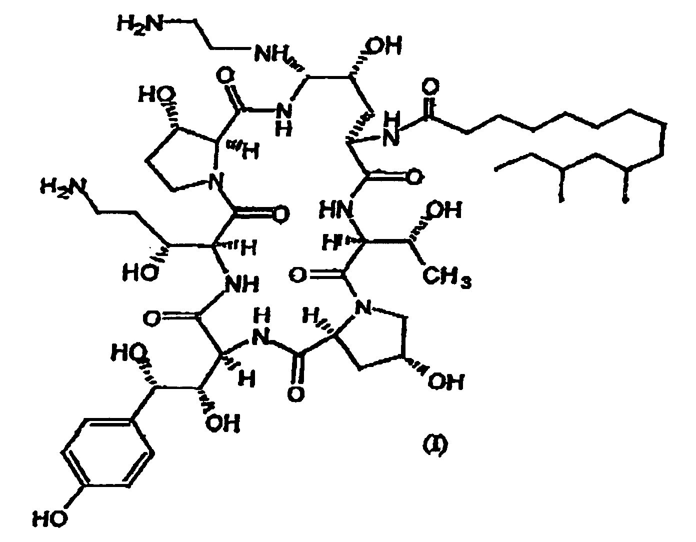 ou un sel pharmaceutiquement acceptable de celui-ci.

2. Utilisation selon la revendication 1, dans laquelle le dérivé de la pneumocandine est formulé pour une administration parentérale.

3. Utilisation selon la revendication 1 ou la revendication 2, dans laquelle l'azole, le polyène ou l'autre agent anti-fongique est formulé pour une administration orale ou parentérale.

4. Utilisation selon l'une quelconque des revendications 1 à 3, dans laquelle l'azole est choisi dans le groupe constitué par le fluconazole, le voriconazole, l'itraconazole, le kétoconazole, le miconazole, ER 30346 et SCH56592 ; le polyène est choisi dans le groupe constitué par l'amphotéricine B, la nystatine ou leurs formes liposomiques et lipidiques ; l'inhibiteur des nucléotides puriques ou pyrimidiques est la flucytosine ; l'inhibiteur du mannane est la predamycine ; l'inhibiteur du facteur d'élongation protéique est la sordarine ou ses analogues ; et la polyoxine est la nikkomycine Z.

5. Utilisation selon l'une quelconque des revendications 1 à 3, dans laquelle l'autre agent anti-fongique est un polyène.

6. Utilisation selon l'une quelconque des revendications 1 à 3, dans laquelle l'autre agent anti-fongique est un azole.

7. Utilisation selon la revendication 5, dans laquelle le polyène est l'amphotéricine B.

8. Utilisation selon la revendication 6, dans laquelle l'azole est le fluconazole.

9. Utilisation selon l'une quelconque des revendications 1 à 8, dans laquelle l'infection est provoquée par un agent pathogène fongique choisi parmi *Cryptococcus spp., Candida spp., Aspergillus spp., Histoplasma spp., Coccidioides spp., Paracoccidioides spp., Blastomyces spp., Fusarium spp., Sporothrix spp., Trichosporon spp., Rhizopus spp., Pseudallescheria spp.,* les dermatophytes, *Paeciliomyces spp., Alternaria* *spp., Curvularia spp., Exophiala spp., Wangiella spp., Pénicillium spp., Saccharomyces spp., Dematiaceous fungii* ou *Pneumocystis carinii*.

10. Utilisation selon la revendication 9, dans laquelle l'agent pathogène fongique est choisi parmi *Cryptococcus spp., Candida spp. ou Aspergillus spp..*

11. Utilisation selon la revendication 7 ou la revendication 8, dans laquelle l'agent pathogène fongique est une souche clinique ou environnementale de *Cryptococcus neoformans*.

12. Utilisation selon la revendication 7, dans laquelle l'agent pathogène fongique est une souche clinique ou environnementale de *Candida albicans* ou *Aspergillus fumigatus*.

13. Utilisation selon la revendication 6, dans laquelle l'azole est SCH 56592.
